# EUROPEAN PATENT APPLICATION

(11) **EP 1 293 776 A2**
(43) Date of publication of application: **19.03.2003**
(21) Application number: 02256274.8
(22) Date of filing: 11.09.2002
(51) Int. Cl.: G01N 33/00, G01N 27/407

(54) **Apparatus for measuring concentration of ammonia gas**

(30) Priority: 11.09.2001 JP 2001274675
(71) Applicant: NGK Spark Plug Company Limited, Nagoya, Aichi (JP)
(72) Inventor: Kitanoya, Shoji, c/o Ngk Spark Plug Co., Ltd, Aichi (JP); Ishida, Noboru, c/o Ngk Spark Plug Co., Ltd, Aichi (JP)
(74) Representative: Brown, Kenneth Richard

(57) **Abstract**

An apparatus for measuring the concentration of ammonia gas, including a solid electrolyte body having oxygen ion conductivity, a reference electrode formed on one surface of the solid electrolyte body and contacting a reference gas, a detecting electrode formed on the other surface of the solid electrolyte body and containing a noble metal and/or a metal oxide therein, a Pd catalyst layer formed on an outer surface of the detecting electrode and including a Pd-containing porous body, and a heater element for heating the solid electrolyte body.

## Description

### BACKGROUND OF THE INVENTION

### Field of the Invention

The present invention relates to an apparatus for measuring the concentration of an ammonia gas, and more particularly to an apparatus for measuring the concentration of an ammonia gas, and capable of selectively measuring the concentration of an ammonia gas in an exhaust gas from an internal combustion engine.

### Related Art

As the regulation of exhaust gas has been tightened in recent years, the development of an apparatus capable of directly detecting and measuring the concentration of an ammonia gas component contained in an exhaust gas from an internal combustion engine, has been required.

Even in, for example, a diesel engine in which an after-treatment unit for an exhaust gas from a catalytic converter and the like has heretofore rarely been provided, the employment of a system provided with a catalytic converter and the like, and adapted to reduce components of noxious gases, such as an inflammable gas in an exhaust gas, is now being discussed.

Since, unlike a gasoline engine, a diesel engine has a high oxygen concentration in an exhaust gas, the contents of inflammable gases, such as CO and HC become low. However, since the content of the air in a gaseous mixture is high, the temperature of a combustion gas lowers, and flame propagation becomes unstable, so that NOx readily occurs. In these circumstances, an attempt is now being made to purify an exhaust gas by using a catalyst for selectively reducing NOx in the exhaust gas.

An exhaust gas from a diesel engine is short of components (CO, HC, etc.) for reducing NOx. Therefore, the introduction of a suitable reducing agent into the as yet unreduced exhaust gas is being discussed as a NOx reduction promoting method. Hydrocarbons are generally used as such reducing agents but, on the other hand, the use of urea is being discussed.

The methods of reducing NOx by using urea include a method of catalytically reducing NOx by utilizing ammonia formed by hydrolyzing urea, and thereby decomposing the resultant product into innoxious N₂ and H₂O. In order to control the quantity of urea to be hydrolyzed, it is necessary to monitor the concentration of excess ammonia gas remaining after the reduction of NOx, and to provide an apparatus for this purpose.

There has been proposed a sensor (refer to, for example, JP-A-60-61654) which is adapted to detect the concentration of a component of an inflammable gas, such as ammonia, on the basis of an electromotive force occurring, in use, between a reference electrode and a detecting electrode which are formed on a surface of an oxygen ion conduction member.

### Problem Addressed by the Invention

Inflammable gases, such as CO, HC exist in an exhaust gas from a diesel engine. A related art gas concentration measuring apparatus has a high sensitivity with respect to inflammable gases other than an ammonia gas. Therefore, it was difficult to measure the concentration of an ammonia gas accurately with a related art gas concentration apparatus. Although an ammonia gas sensor using a zeolite film coated on a digital capacitor has been proposed for this use, a reliable one has not yet been developed. In short, an apparatus capable of reliably and selectively detecting an ammonia gas, and speedily measuring the concentration thereof has not heretofore been available.

### SUMMARY OF THE INVENTION

The present invention has been made in view of the above-mentioned circumstances, and has an object to provide an ammonia gas concentration sensor capable of measuring the concentration of an ammonia gas speedily and accurately.

The present invention also has an object to provide an ammonia gas sensor capable of detecting an ammonia gas selectively.

The present invention provides in one aspect an apparatus for measuring the concentration of an ammonia gas, the apparatus comprising: a solid electrolyte body having an oxygen ion conductivity, a reference electrode formed on one surface of the solid electrolyte body for contacting a reference gas, a detecting electrode formed on the other surface of the solid electrolyte body and containing at least one of a noble metal and/or a metal oxide therein, and a Pd catalyst layer formed on an outer surface of the detecting electrode and including a Pd-containing porous body.

The present invention further provides in another aspect an apparatus for measuring the concentration of an ammonia gas, the apparatus comprising: a solid electrolyte body having an oxygen ion conductivity, a reference electrode formed on one surface of the solid electrolyte body for contacting a reference gas, a detecting electrode formed on the other surface of the solid electrolyte body and containing at least one of a noble metal and/or metal oxide therein, and a Pd catalyst layer formed on an outer surface of the detecting electrode and including a porous body and Pd supported by said porous body.

A best performance of selective ammonia detection in the measurement gas is attained when the detecting element formed on the oxygen ion-conducting solid electrolyte body is made of a mixture of Pt and Au and ZrO₂, the porous body is made of porous spinel and the porous Pd is formed on the porous spinel. The metal oxide such as ZrO₂, Al₂O₃ and TiO₂ in the detecting electrode reduces interference with ammonia detection when the measurement gas contains oxygen.

In these apparatuses for measuring the concentration of an ammonia gas, it is preferable to provide a heater element for heating the solid electrolyte body.

Also, it is preferable that the detecting electrode be formed on only a portion of said other surface of the solid electrolyte body which corresponds to a heating resistor formed in the interior of the heater element.

Also preferably, the solid electrolyte body has a closed cylindrical shape (i.e. tubular shape having a bottom), and the detecting electrode is formed on only a portion of an outer surface of the cylindrical solid electrolyte body, which detecting electrode extends from the position corresponding to the region of an interface between a heating resistor provided in the interior of the heater element that is disposed close to a closed end portion of the cylindrical solid electrolyte body.

Also, it is preferable that a temperature control unit adapted to control a voltage applied to the heater element on the basis of internal resistance of the solid electrolyte body, be provided.

Also, it is preferable that an internal resistance measuring electrode be provided which is formed on said one surface of the solid electrolyte body and in the vicinity of the heating resistor of the heater element so as to be separated from the reference electrode, and which is adapted to measure internal resistance of the solid electrolyte body, and that the temperature control unit be adapted to measure the internal resistance of the solid electrolyte body by the internal resistance measuring electrode and detecting electrode.

Embodiments of the invention will now be described by way of example with reference to the accompanying drawings.

### BRIEF DESCRIPTION OF THE DRAWINGS

Fig. 1 is a sectional view schematically showing the construction of an apparatus for measuring the concentration of an ammonia gas according to a first embodiment of the present invention.
Fig. 2 is a graph showing the correlation between a sensor output and the concentration of a gas to be detected in the apparatus according to the first embodiment of the present invention.
Fig. 3 is a graph showing the correlation between a sensor output and the concentration of a gas to be detected in an apparatus for measuring the concentration of an ammonia gas according to a comparative example.
Fig. 4 is a graph showing a sensitivity ratio of a gas to be detected with respect to an ammonia gas in the apparatuses according to the first embodiment and comparative example.
Fig. 5 is a sectional view schematically showing the construction of the apparatus for measuring the concentration of an ammonia gas according to a second embodiment of the present invention.
Fig. 6 is a sectional view schematically showing the construction of the apparatus for measuring the concentration of an ammonia gas according to a third embodiment of the present invention.

### DESCRIPTION OF THE PREFERRED EMBODIMENTS

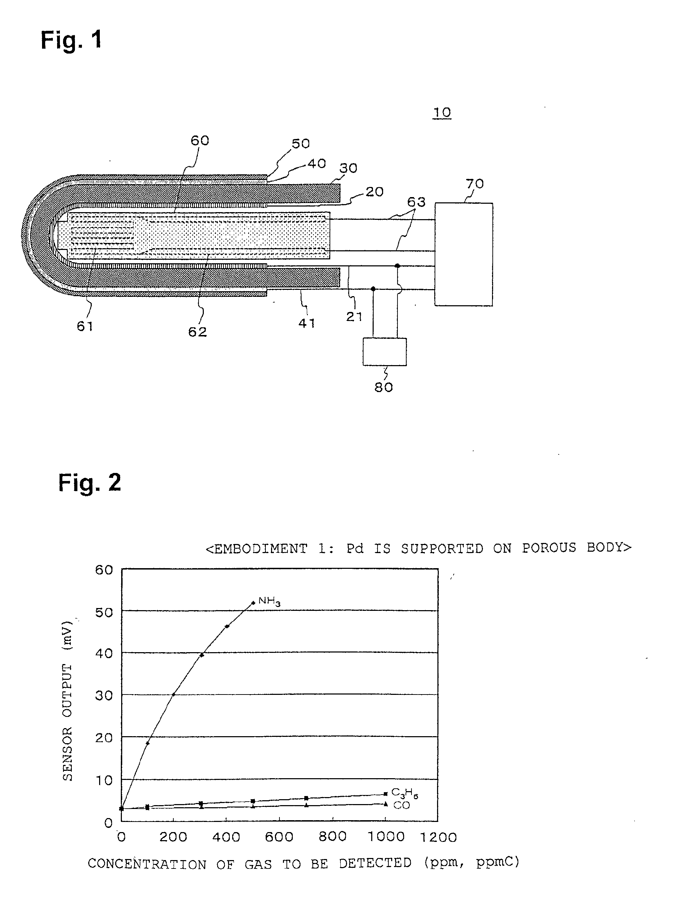

With reference to Fig. 1, an apparatus 10 for measuring the concentration of an ammonia gas is provided with a solid electrolyte body 30 having an oxygen ion conductivity, a reference electrode 20 formed on one surface of the solid electrolyte body and contacting a reference gas, a detecting electrode 40 formed on the other surface of the solid electrolyte body and containing a noble metal or a metal oxide therein, a Pd catalyst layer 50 formed on an outer surface of the detecting electrode and including a Pd-containing porous body, and a heater element 60. Using this apparatus, it is possible to burn inflammable gases other than an ammonia gas on the Pd catalyst layer, selectively detect and measure the concentration of an ammonia gas, and improve the detecting and measuring accuracy. Therefore, when the detecting electrode is exposed to an ammonia gas, a potential difference occurs between the detecting electrode and the reference electrode, and a sensor output is measured in accordance with this potential difference. This enables the concentration of the ammonia gas to be measured on the basis of the measured sensor output and preset correlation.

The apparatus 10 also includes a heater element 60, a temperature control unit 70 and a sensor output measuring unit 80.

The reference electrode 20 is an electrode layer formed on an inner surface of the solid electrolyte body 30 including an inner bottom surface thereof, and made of a metal, such as Pt exposed to a reference gas. The reference electrode 20 is electrically connected to the temperature control unit 70 and sensor output measuring unit 80 via a reference electrode lead wire 21.

The solid electrolyte body 30 is made of a material having oxygen ion conductivity, such as zirconia sinter and LaGaO₃ sinter, and which has a closed cylindrical form and includes a curved bottom surface. This solid electrolyte body is provided with the reference electrode 20 on an inner surface thereof including an inner bottom surface thereof, and detecting electrode 40 on an outer surface thereof including an outer bottom surface thereof. This solid electrolyte body is exposed to view at a portion thereof which is in the vicinity of an end surface thereof including that end surface.

The detecting electrode 40 is an electrode layer formed on an outer surface of the solid electrolyte body 30 including an outer bottom surface thereof for exposure to a gas to be measured. This detecting electrode contains one or no less than two metals (especially, noble metals) and one or not smaller than two metal oxides therein, and is electrically connected to the temperature control unit 70 and sensor output measuring unit 80 via a detecting electrode lead wire 41. The Pd catalyst layer 50 is formed on an outer surface of the detecting electrode 40. The metals used in the detecting electrode 40 are Pt, Au, etc., and the metal oxides used are oxides, such as zirconia, alumina and titania.

The Pd catalyst layer 50 is a catalyst layer supported on the detecting electrode 40 and in use is exposed to a gas to be measured; it is made of a Pd-supporting or a Pd-containing porous body. The Pd burns or oxidizes inflammable gases other than an ammonia gas. The porous body used in the Pd catalyst layer 50 meets the purpose as long as it is capable of allowing an inflammable gas, which is contained in a gas to be measured, to flow to the detecting electrode, and, for example, spinel or alumina and the like are used. The porous body is capable of maintaining at a substantially constant level a velocity of flow at its outer surface and a flow rate of the gas to be measured which flows from that surface to the detecting electrode, irrespective of the velocity of flow and flow rate of the same gas flowing on the mentioned surface. Accordingly, the porous body is capable of lowering the dependency of the gas to be measured upon the velocity of flow and flow rate of the gas. The porous body also acts both as a protective layer with respect to the poisoning of the detecting electrode and as a reinforcing layer and the like for increasing the strength thereof. The porous body may have two or more layers.

The heater element 60 is an element for heating the solid electrolyte body 30, and maintains the temperature of the heated solid electrolyte body at a predetermined level. The heater element 60 has a heating resistor 61 buried in the portion thereof which is in the vicinity of a front end thereof, and a heating resistor lead wire 62 for supplying electric power to the heating resistor 61, and is electrically connected to the temperature control unit 70 via a heater element lead wire 63. The heater element 60 is of rod-like and flat plate-like shapes, and disposed so that the front end portion thereof contacts the interior of the closed cylindrical solid electrolyte body 30. The front end portion of the heater element 60 may have a curved surface similar to the inner bottom surface of the solid electrolyte body 30.

The temperature control unit 70 is a device for controlling the temperature by regulating a voltage applied to the heater element on the basis of internal resistance of the solid electrolyte body 30, and includes a device for measuring the internal resistance of the solid electrolyte body 30, and a device for controlling a voltage applied to the heater element on the basis of this internal resistance. The methods of measuring the internal resistance of the solid electrolyte body 30 include a method of measuring the resistance between the reference electrode and detecting electrode, and a method of measuring resistance between an internal resistance measuring electrode, which is formed separately from the reference electrode, on an inner surface of the solid electrolyte body 30 and the detecting electrode. The temperature control unit 70 is electrically connected to each of the reference electrode 20, detecting electrode 40 and heater element 60 via lead wires 21, 41, 63. Owing to the provision of the temperature control unit 70, sharp detection and accurate measurement of the concentration of an object gas become possible.

The sensor output measuring unit 80 is a unit for measuring a sensor output on the basis of a potential difference between the reference electrode 20 and detecting electrode 40, and is electrically connected to each of the reference electrode 20 and detecting electrode 40 via the lead wires 21, 41.

An example of the manufacturing of the apparatus for measuring the concentration of an ammonia gas according to the first embodiment will now be described.

First, in the manufacturing of detecting electrode paste used for the production of the detecting electrode 40 shown in Fig. 1, each metal powder is mixed at a predetermined ratio (for example, 90 wt% of Pt and 10 wt% of Au), and a predetermined quantity of ZrO₂ is then added to the resultant mixture. Then ethylcellulose as a binder and butyl carbitol as a solvent are added to and mixed with the resultant product. Thus, detecting electrode paste can be obtained.

The manufacturing of the closed cylindrical sensor element is done in the following manner. A powder of 4.5 mol% of Y₂O₃-containing yttria-stabilized zirconia (which will hereinafter be referred to simply as YSZ) is packed in a closed cylindrical rubber mold, and pressure molded. Paste forming the detecting electrode lead wire is then printed on an outer surface of the closed cylindrical molded body obtained, and the resultant product is calcined to obtain a closed cylindrical solid electrolyte body on which the detecting electrode lead wire is provided. The whole of an inner surface of the solid electrolyte body is then plated with platinum to form a reference electrode. The detecting electrode paste prepared in advance is then applied to a relative portion, and the resultant product is burnt in atmospheric air at 1400°C for 1 hour to form a detecting electrode. Then, spinel is flame sprayed on an outer surface of the detecting electrode to form a porous layer. A sensor element on which the porous layer is formed is then immersed in a palladium nitrate solution of a predetermined concentration (0.01 to 0.2 mol/L), and the resultant product is dried, and then burned in atmospheric air at 800°C for 10 minutes, to form a porous Pd catalyst layer. A heater element is then set so that a front end portion thereof contacts an inner bottom surface of the solid electrolyte body. Finally, the reference electrode lead wire, detecting electrode lead wire and heater element lead wire are connected to the temperature control unit to obtain an apparatus for measuring the concentration of an ammonia gas.

The sensor output in the first embodiment (having a Pd catalyst) and that in a comparative example (not having a Pd catalyst) will now be comparatively described.

The apparatus for measuring the concentration of an ammonia gas according to the first embodiment is as shown in Fig. 1. The apparatus of a comparative example is as shown in Fig. 1 except that the catalyst layer is made of a non-Pd-containing porous body alone. The methods of manufacturing the apparatuses of the first embodiment and comparative example are identical with each other except the Pd-supporting step, and the sizes of these apparatuses are the same.

The sensor characteristics tests will first be described. Model gas units formed by imitating an exhaust gas unit in an actual vehicle were used, and a closed-end portion of the apparatus of the first embodiment or an apparatus of the comparative example was disposed in an intermediate portion of a flow passage. The temperature of the heater element in each apparatus was set to 600°C, and a gas to be measured (a base gas and a gas to be detected) containing one kind of gas to be detected (of a predetermined concentration) out of NH₃, CO, C₃H₆ was made to flow at 190°C and a flow rate of 15L/min. The sensor outputs during the tests were measured. A summary of the measuring conditions is shown in Table 1. The portion of each of the model gas units which is on the side of the reference electrode of the gas concentration measuring apparatus is exposed to the atmospheric air, and the portion which is on the side of the detecting electrode is exposed to the gas to be measured. The balance of N₂ in Table 1 means remaining gas composition occurring when one component of a gas to be measured is added to a base gas except N₂.

**Table 1**

| Composition of the gas to be measured | Base gas | O₂=10%, CO₂=7%, H₂O=7%, N₂=balance | |
|---|---|---|---|
| | Gas to be detected | NH₃[ppm] | 0, 200, 400, 600, 800, 1000 |
| | | CO[ppm] | 200, 300, 500, 700, 1000 |
| | | C₃H₆[ppmC] | 200, 300, 500, 700, 1000 |
| Temperature of gas | 190°C | | |
| Flow rate of gas | 15 L/min | | |
| Temperature of element | 600°C | | |

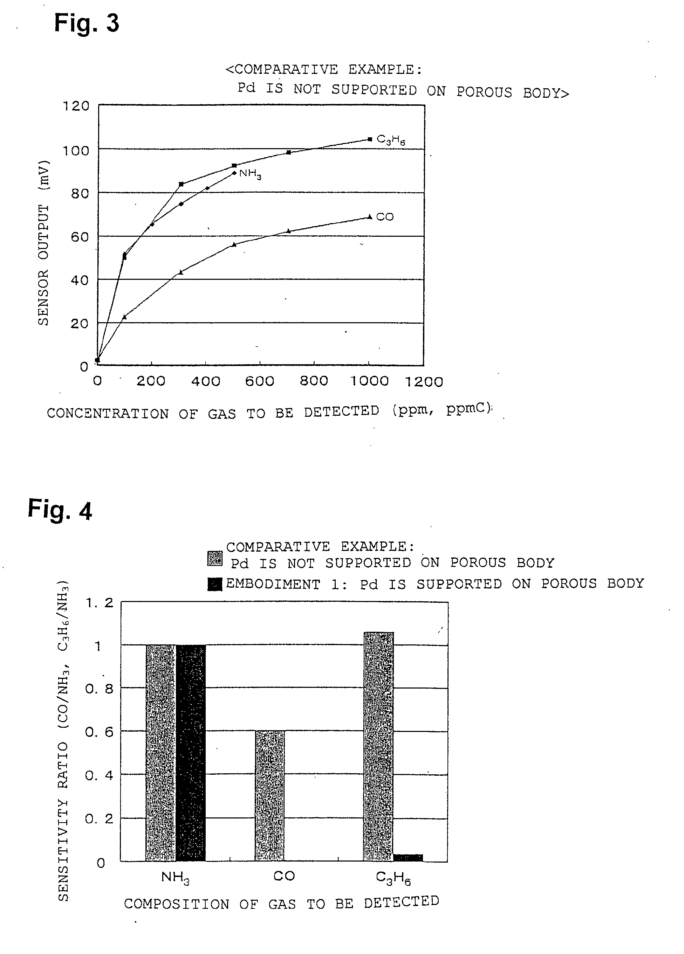

The results of the sensor characteristics tests will be described with reference to the drawings. Fig. 2 is a graph showing the correlation between a sensor output and the concentration of the gas to be detected in the apparatus according to the first embodiment of the present invention. Fig. 3 is a graph showing the correlation between a sensor output and the concentration of the gas to be detected in the apparatus according to the comparative example. Fig. 4 is a graph showing a sensitivity ratio of the gas to be detected to the ammonia gas in the apparatuses according to the first embodiment and comparative example.

Referring to Fig. 3 and Fig. 4, in the comparative example in which Pd is not supported on the porous body, all of the components of NH₃, CO and C₃H₆ have a high sensitivity and, moreover, the correlation between the sensor outputs and gas concentration with respect to NH₃ and the correlation therebetween with respect to CO are similar to each other. On the whole, the selectivity of gas components was not recognized.

Referring to Fig. 2 and Fig. 4, the sensor outputs with respect to CO and C₃H₆ are held down to a level lower than 20 mV at all concentrations in the first embodiment in which Pd is supported on the porous body. Although the sensor output with respect to NH₃ somewhat lowers as compared with that in the comparative example, it increases with an increase in the gas concentration to a level higher than those with respect to CO and C₃H₆. In short, it is understood that the selectivity of NH₃ is improved noticeably owing to the supporting of Pd on the porous body.

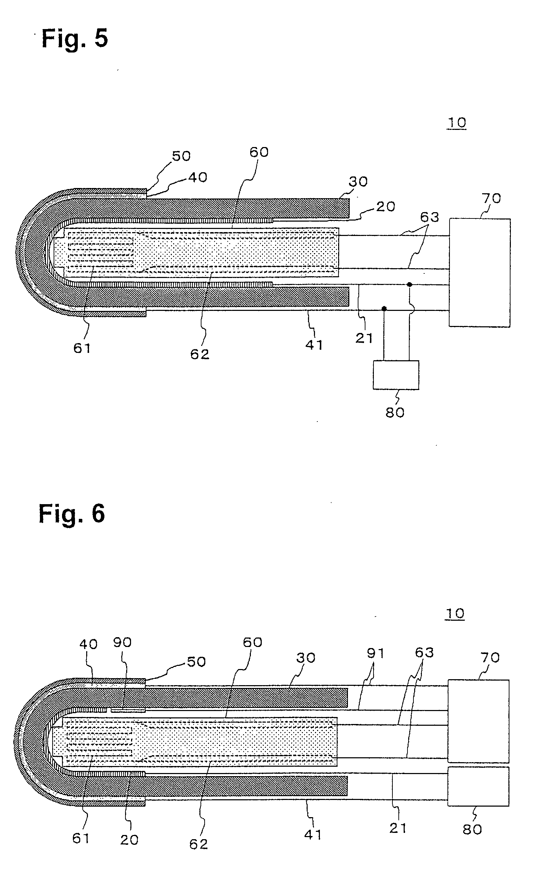

Referring to Fig. 5 showing the second embodiment, the same detecting electrode 40 as in the apparatus of the first embodiment is preferably provided only on the portion of an outer surface of a solid electrolyte body 30 that corresponds to a heating resistor 61 formed in the interior of a heater element 60, or, stated differently, only the portion of that outer surface that extends from the position corresponding to the neighborhood of an interface between the heating resistor 61 within the heater element 60 and a lead portion 62 of the heating resistor, to the position on that surface which corresponds to a front end portion of the solid electrolyte body. The reason is that, when the electrode is formed on only that portion of the outer surface of the solid electrolyte body which is maintained at a high, uniform and stable temperature, the dependency of the sensor output upon the temperature can be reduced.

Referring to Fig. 6 showing the third embodiment, it is preferable to form in the same apparatus as in the first and second embodiments an internal resistance measuring electrode 90 separately from and independently of a reference electrode 20 and on the portion of an inner surface of the solid electrolyte 30 which is in the vicinity of the heating resistor. In this third embodiment the internal resistance measuring electrode 90 and the detecting electrode 40 are electrically connected via internal resistance measuring lead wires 91 to the temperature control unit 70, and thereby measures the resistance between the internal resistance measuring electrode 90 and the detecting electrode 40, and a detecting electrode lead wire 41 and a reference electrode lead wire 21 are connected to only a sensor output measuring unit 80 and not to the temperature control unit 70. Owing to this arrangement, the measurement of the internal resistance of the solid electrolyte body and that of a sensor output are conducted by using different lead wires. Therefore, it becomes possible to conduct accurate measurement of the internal resistance of the solid electrolyte body, a precise temperature control operation, and accurate measurement of a sensor output.

According to the present invention, therefore, an ammonia gas contained in a gas to be measured can be detected selectively and sharply, and the concentration thereof can be measured speedily and accurately.

## Claims

1. An apparatus for measuring the concentration of an ammonia gas, **characterized in that** the apparatus is provided with:
a solid electrolyte body (30) having an oxygen ion conductivity,
a reference electrode (20) formed on one surface of the solid electrolyte body for contacting a reference gas,
a detecting electrode (40) formed on the other surface of the solid electrolyte body and containing at least one of a noble metal and a metal oxide therein, and
a Pd catalyst layer formed on an outer surface of the detecting electrode and including a porous body and Pd.

2. An apparatus according to claim 1, wherein in said Pd catalyst layer the Pd is contained within the porous body.

3. An apparatus according to claim 1 wherein in said Pd catalyst layer the Pd is supported on said porous body.

4. An apparatus according to any of Claims 1 to 3, further comprising a heater element (60) for heating the solid electrolyte body.

5. An apparatus according to Claim 4, wherein the detecting electrode (40) is formed on only a portion of said other surface of the solid electrolyte body that corresponds to a heating resistor (61) provided in the interior of the heater element (60).

6. An apparatus according to Claim 4 or Claim 5, wherein:
the solid electrolyte body (30) is of a closed cylindrical shape,
the detecting electrode (40) is formed on only the portion of said other surface of the solid electrolyte body that extends from the position on the same surface corresponding to the region of an interface between the heating resistor (61) formed in the interior of the heater element and a lead portion (62) of the heating resistor to the position on the same surface which corresponds to a closed end portion of the solid electrolyte body.

7. An apparatus according to any of Claims 4 to 6, wherein the apparatus is provided with a temperature control unit (70) adapted to control a voltage applied to the heater element, on the basis of internal resistance of the solid electrolyte body.

8. An apparatus according to Claim 7, wherein there is provided an internal resistance measuring electrode (90) which is formed on said one surface of the solid electrolyte body and in the vicinity of a heating resistor (61) of the heater element (60) so as to be separated from the reference electrode (20), and which is adapted to measure internal resistance of the solid electrolyte body,
the temperature control unit (70) being adapted to measure the internal resistance of the solid electrolyte body by the internal resistance measuring electrode and detecting electrode.

9. An apparatus according to any preceding claim wherein the detecting electrode (40) contains a mixture of at least one noble metal and at least one metal oxide.
